Europäisches Patentamt

European Patent Office

Office européen des brevets

(11) Publication number: **0 287 380**
**A2**

## (12) EUROPEAN PATENT APPLICATION

(21) Application number: **88303389.6**

(22) Date of filing: **14.04.88**

(51) Int. Cl.⁴: **C 07 C 2/78**
**C 07 C 11/24, B 01 J 19/24**

(30) Priority: **16.04.87 GB 8709265**

(43) Date of publication of application:
**19.10.88 Bulietin 88/42**

(84) Designated Contracting States:
**BE DE ES FR GB IT NL**

(71) Applicant: **The British Petroleum Company p.l.c.**
**Britannic House Moor Lane**
**London EC2Y 9BU (GB)**

(72) Inventor: **Howard, Mark Julian**
**The British Petroleum Co. p.l.c. Chertsey Road**
**Sunbury-on-Thames Middlesex TW16 7LN (GB)**

(74) Representative: **Dodding, Robert Anthony et al**
**BP INTERNATIONAL LIMITED Patents Division Chertsey**
**Road**
**Sunbury-on-Thames Middlesex, TW16 7LN (GB)**

(54) Burner for conversion process.

㊙ A gas conversion reactor for converting gaseous saturated hydrocarbons into unsaturated hydrocarbons is supplied with pre-mixed reactants comprising a saturated hydrocarbon gas such as methane and an oxygen containing gas, the reactants being fuel rich. The reactor has an annular or tubular inlet zone having a common wall with an annular outlet zone thereby allowing indirect heat exchange between the products in the outlet zone and the reactants in the inlet zone. The cross sectional area of the outlet zone is preferably greater than that of the inlet zone. The reactants are partially oxidised in the reactor and the emerging products are quenched.

FIG.1

FIG.2

Bundesdruckerei Berlin

EP 0 287 380 A2

**Description**

## BURNER FOR CONVERSION PROCESS

The present invention relates to a conversion process and more particularly relates to a gas conversion reactor and a gas conversion process for producing useful products such as acetylene.

It is known to produce acetylene by partial oxidation of gaseous or vaporised hydrocarbons with oxygen by preheating the reactants together or separately, supplying the mixture to a reaction chamber, reacting the mixture and rapidly cooling or quenching the reaction gases so as to give maximum yield of acetylene. The quenching is generally effected by spraying water into the reaction products. Examples of apparatus and processes for producing unsaturated hydrocarbons are disclosed in GB patent numbers 821856, 834419 and 876261.

The present invention relates to an improved process for producing unsaturated hydrocarbons such as acetylene, ethylene and higher hydrocarbons which alleviates or eliminates some of the problems associated with the prior art processes.

Thus according to the present invention there is provided a process for producing unsaturated hydrocarbons in which (a) pre-mixed saturated hydrocarbon gas and an oxygen containing gas having a stoichiometric ratio in excess of that required for complete combustion is passed into a reaction chamber, (b) the reaction chamber being adapted to allow indirect heat exchange between the reactants and the reaction products, (c) the hydrocarbon gas and oxygen containing gas are ignited and reacted together and (d) quenching the reaction products are quenched.

According to a further aspect of the invention there is provided a gas conversion reactor comprising a reaction chamber having an inlet zone leading to an outlet zone the zones being arranged to allow indirect heat exchange from the outlet zone to the inlet zone the inlet zone having an inlet for pre-mixed reactant gases and the reaction chamber having an outlet to a quenching zone.

It is preferred that the cross sectional area of the outlet zone is greater than the inlet zone. For example the outlet zone may initially at the upstream end have the same or a similar cross sectional area as the inlet zone but then diverges along its length. Alternatively the outlet zone may have one or more steps, each step being of the same or of greater cross sectional area than the inlet zone. Other embodiments to achieve the wider outlet zone than inlet zone may be readily envisaged.

In one embodiment of the present invention, the reaction chamber has an inlet zone in the form of a central annulus or tube leading to an outlet zone in the form of an annular cylinder or chamber having a common wall with the inlet zone thereby allowing indirect heat exchange to occur. The reactant gases pass along the inlet zone and turn back to enter the outlet zone where reaction occurs, the common wall allowing heat exchange of products and reactants to occur.

In an alternative embodiment of the invention, the reaction chamber may take the form of an interconnected planar array of inlet and outlet zones thereby maintaining the axial flow profile of gases through the reactor. The planar form of reactor may facilitate a relatively increased throughput capacity and allow relatively straightforward scale up capability.

Allowing indirect heat exchange between the reactants and the reaction products appears to enable fuel rich gas mixtures, having compositions which are beyond the rich limit of flammability, to be reacted together.

The reactor may be made from a variety of materials that are capable of withstanding the reaction conditions. Thus the burner may be fabricated from quartz or metal and ceramic materials such as zirconia, alumina or silicon carbide lend themselves to this form of construction.

The reactor may comprise a single reaction chamber or may comprise a multiplicity of reaction chambers, the reaction chambers being arranged in a matrix.

The reactor may be operated at below atmospheric pressure, atmospheric pressure or at elevated pressure. It is envisaged that the products of the reactor will be dependent on reactor conditions e.g. for partial oxidation of methane at elevated pressure the formation of ethylene may be more favoured than acetylene with an increased possibility of higher hydrocarbon formation.

The saturated hydrocarbon is preferably gaseous at ambient temperatures, suitable compounds being methane, ethane, propane and butane. The oxygen containing gas is preferably air, oxygen enriched air or pure oxygen.

The reactants may be pre-heated and/or pre-mixed prior to entry to the reaction chamber but it is a feature of the present invention that normally a pre-heat will not be necessary. This reduces the necessity for pre-heating hydrocarbon and oxygen at high temperatures which is an inherently hazardous procedure.

The reaction products are preferably quenched by spraying water into them as they emerge from the reaction chamber or by any suitable method allowing rapidly heat removal without product degradation. Alternative methods of quenching include rapid gas expansion, introduction of an inert gas such as nitrogen or helium into the reaction products or by use of an active hydrocarbon quench ranging from use of $C_2$-$C_4$'s to gasoline fractions to heavy oil. Partial cracking of the quenched hydrocarbon may enhance the yield of desired products such as acetylene and ethylene.

The exit gas temperatures are of the order 1250° C, a value which is substantially lower than for many prior art reactors (usually of the order 1500° C). This enables a less severe quench to be used while preserving the yield of acetylene product.

Hydrogen gas may be fed with the reactants into the reaction chamber. This procedure usually enables enhanced yields of acetylene to be obtained. Also other gases such as carbon dioxide, water/steam or carbon monoxide may be fed with the reactants to produce particular products. Inert diluents such as nitrogen, helium etc may be added if required.

The ability of the reactor to allow heat exchange between reactants and both the reaction products and reaction zone enables an inherent pre-heat of the feed to occur simultaneously with a lower exhaust or reaction product temperature.

The invention will now be described by way of example only and with reference to Figures 1 to 5 of the accompanying drawings.

Figure 1 shows a vertical section through a reactor for producing unsaturated hydrocarbons according to the present invention and having a stepped outlet zone of greater cross sectional area than the inlet zone. Figure 2 shows a horizontal section along line II - II of Figure 1.

Figure 3 shows a vertical section through a reactor for producing unsaturated hydrocarbons according to the present invention and having a divergent outlet zone of greater cross-sectional area than the inlet zone. Figure 4 shows a horizontal section along line IV - IV of Figure 3.

Figure 5 shows a schematic representation of part of a planar form of reactor according to the present invention and showing three of the reaction chambers.

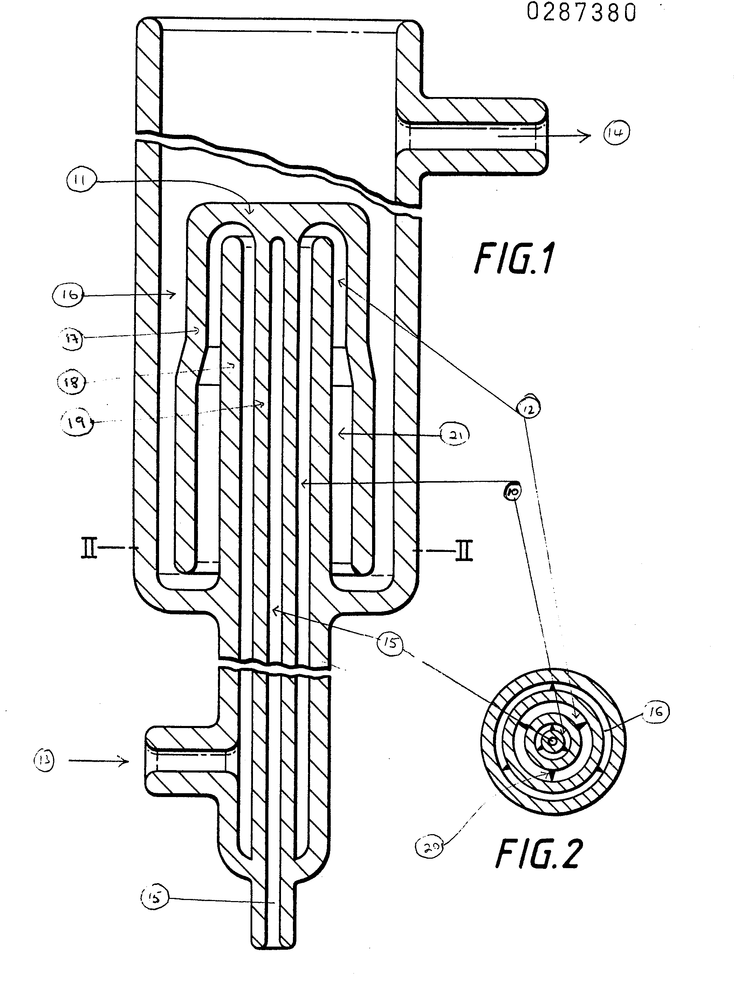

In Figure 1, the reactor comprises an inlet zone 10, a reaction chamber 11 and an outlet zone 12. The reaction chamber 11 is in the form of a cylinder having an inlet 13 and an outlet 14 for the reactants and products respectively. The chamber has three annular sections 10, 12, 16 with internal annular walls 17, 18, 19. The inlet zone is located in the inner annulus 10 and communicates with the inlet 13 (for reactor feed) and with the outer annulus 12. The downstream end of the outer annulus 12 has a stepped outlet zone 21 of greater cross sectional area than the inlet zone. The inner and outer annulus communicate at the end of the cylinder remote from the inlet. This arrangement causes the inlet gases to follow a tortuous path within the chamber and to be pre-heated prior to reaching the outer annulus reaction zone. The annular walls are separated by a number of internal projections or 'pips' which are sometimes also useful in assisting stabilisation of the reaction.

The inlet lies upstream of the inlet zone 10 and takes the form of a side arm entry for the reactants. The outlet 14 receives the products of the reaction from the outer annulus and quenching occurs due to the rapid heat loss to the surroundings causing quench and product cooling and enabling sampling of the reaction products. The reaction chamber may have a central tube 15 for receiving a thermocouple.

In use of the reactor, pre-mixed methane and oxygen are passed into the reaction chamber 11 through the side arm 13 communicating with the inner annulus or inlet zone 10. The reactant gases pass through the inlet and outlet zones and emerge through outer annulus 16 into the volume above the reaction chamber 11. The reactant gases are then ignited by suitable means and the resultant flame is then lowered by control of fuel gas/oxygen stoichiometry and flow rate until the flame rests near the outlet end of the stepped outlet zone. In an optimised burner operation, the flame can be lowered to just above the top of the reaction chamber. Then as heat transfer occurs to the cold feed the flame is drawn into the outlet recuperative zone without further adjustment. The stoichiometry of the gas feed then can be changed to the desired fuel rich ratio. The pre-mixed gases may be pre-heated if desired. The design of the reactor chamber, flow rates, temperatures etc are chosen so as to enable a reaction zone to develop in the central annulus of the reaction chamber. The products of the reaction exit by the outer annulus into the outlet zone, typical exit gas temperatures observed being of the order 1250°C. The residence times of the reactant gases within the reaction chamber depend upon the temperature profile. Short residence times (of the order less than 20 to 40 msec) are advantageous for $C_2$ production.

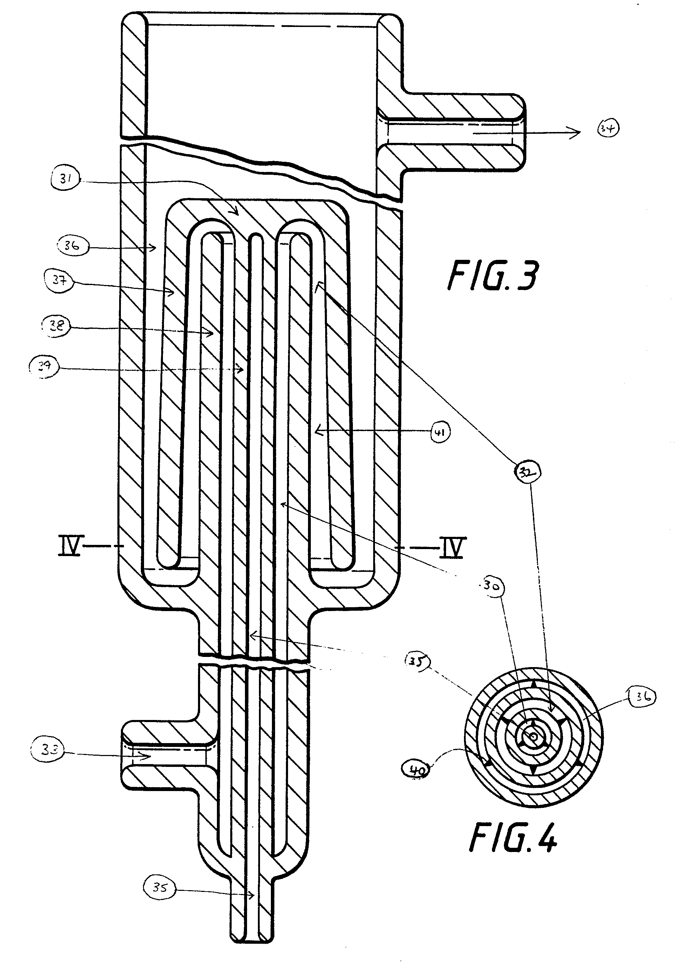

Figures 3 and 4 show an alternative design of reactor having a divergent outlet zone. In Figure 3, the reactor comprises an inlet zone 30, a reaction chamber 31 and an outlet zone 32. The reaction chamber 31 is in the form of a cylinder having an inlet 33 and an outlet 34 for the reactants and products respectively. The chamber has three annular sections 30, 32, 36 with internal annular walls 37, 38, 39. The inlet zone is located in the inner annulus 30 and communicates with the inlet 33 (for reactor feed) and with the outer annulus 32. The downstream end of the outer annulus 32 has a divergent outlet zone 41 of greater cross sectional area than the inlet zone. The inner and outer annulus communicate at the end of the cylinder remote from the inlet. This arrangement causes the inlet gases to follow a tortuous path within the chamber and to be pre-heated prior to reaching the outer annulus reaction zone. The annular walls are separated by a number of internal projections or 'pips' which are sometimes also useful in assisting stabilisation of the reaction.

The inlet lies upstream of the inlet zone 30 and takes the form of a side arm entry for the reactants. The outlet 34 receives the products of the reaction from the outer annulus and quenching occurs due to the rapid heat loss to the surroundings causing quench and product cooling and enabling sampling of the reaction products. The reaction chamber may have a central tube 35 for receiving a thermocouple. The mode of operation of the reactor is similar to that of the stepped outlet zone reactor described above.

## Table 1
## Partial Combustion of Methane in a Reactor

| Reactor Type | Feed Composition % vol | | | | Flow nlmin$^{-1}$ | CH$_4$ Conversion (%) | Product Selectivities % C mol | | | | |
|---|---|---|---|---|---|---|---|---|---|---|---|
| | CH$_4$ | H$_2$ | O$_2$ | N$_2$ | | | CO | CO$_2$ | C$_2$H$_2$ | C$_2$H$_4$ | C$_3$-C$_7$ |
| Figure 1 | 55.5 | – | 35 | 9.5 | 12.1 | 96 | 55.8 | 7.2 | 32 | 3.2 | 1.8 |
| Figure 1 | 59.5 | – | 33 | 7.5 | 14.8 | 91 | 48.9 | 5.4 | 40 | 2.7 | 3.0 |
| Figure 1 | 60.5 | – | 32.5 | 7 | 15.2 | 88 | 47.9 | 5.1 | 41 | 2.4 | 3.6 |
| Figure 1 | 60.5 | – | 32.5 | 7 | 13.2 | 90 | 48.0 | 5.0 | 41 | 2.3 | 3.7 |
| Figure 1 | 59.5 | – | 31.5 | 9 | 8.9 | 86 | 48.7 | 4.7 | 40 | 2.7 | 3.9 |
| Figure 1 | 61 | – | 32 | 7 | 14.0 | 89 | 47.5 | 5.0 | 42 | 2.3 | 3.2 |
| Figure 1 | 61 | – | 32 | 7 | 15.3 | 87 | 48.5 | 5.2 | 40 | 2.6 | 3.7 |
| Figure 1 | 61.5 | – | 32 | 6.5 | 15.3 | 85 | 49.0 | 5.1 | 39 | 2.7 | 4.2 |
| Figure 1 | 62.0 | – | 31.5 | 6.5 | 14.3 | 85 | 47.9 | 4.4 | 41 | 2.5 | 4.2 |
| | | | | | | | | | | | |
| Figure 3 | 60 | – | 32 | 7 | 16.9 | 92 | 49.4 | 5.4 | 39 | 3.0 | 3.2 |
| Figure 3 | 55 | 8 | 30.5 | 6.5 | 18.9 | 89 | 47.4 | 5.3 | 41 | 3.1 | 3.2 |
| Figure 3 | 51 | 15.5 | 28 | 5.5 | 20.0 | 87 | 45.9 | 5.0 | 42 | 3.5 | 3.6 |
| | | | | | | | | | | | |
| Similar inlet & outlet zones | 57.6 | – | 35.6 | 6.8 | 6.35 | 92.3 | 54.3 | 6.6 | 34 | 2.7 | 2.4 |
| | 47.7 | 17.6 | 29.9 | 4.8 | 18.1 | 90.9 | 49.9 | 5.3 | 41 | 2.2 | 2.4 |
| | 36.2 | 35.6 | 20.2 | 8.1 | 26.6 | 69.9 | 44.5 | 3.6 | 43 | 4.8 | 4.1 |

<center>Table 2</center>
<center>Partial Combustion of Ethane in a Reactor</center>

| Total Flow | $C_2H_6$ | $H_2$ | $N_2$ | Conversion | Selectivities % C-Mol | | | | | | | | $C_{2+}$ Yield % C-mol/mol | |
| | $O_2$ | $O_2$ | $O_2$ | | | | | | | | | | | |
| nl min$^{-1}$ | | | | % mol | $C_2H_4$ | $C_2H_2$ | $CH_4$ | CO | $CO_2$ | $C_3/C_4$ | Arom. | Soot | $C_2H_6$ | $O_2$ |
|---|---|---|---|---|---|---|---|---|---|---|---|---|---|---|
| 6.3 | 1.52 | 0 | 0.47 | 98.1 | 43.1 | 9.3 | 11.0 | 32.0 | 1.62 | 2.42 | 0.62 | 0 | 54.4 | 82.6 |
| 6.2 | 1.65 | 0 | 0.51 | 97.1 | 49.0 | 7.0 | 10.3 | 29.3 | 1.36 | 2.67 | 0.45 | 0 | 57.4 | 94.7 |
| 7.4 | 1.77 | 0 | 0.52 | 96.1 | 53.4 | 5.7 | 9.8 | 26.5 | 1.12 | 2.96 | 0.49 | 0 | 60.2 | 106.5 |

Tables 1 and 2 show results obtained using reactors according to the present invention. Table 1 shows the partial combustion of methane in a reactor of the type shown in Figures 1 and 3 and also in a reactor having similar cross sectional areas of inlet and outlet zones. Table 2 shows results for the partial combustion of ethane in a reactor having similar cross sectional areas of inlet and outlet zones.

The tables show the higher yields of acetylene obtained from the stepped reactors i.e. the reactors having outlet zones of increased cross sectional area relative to the inlet zone. The use of hydrogen co-feed reduces conversion and improves the selectivity to higher hydrocarbons whilst leaving yields relatively unchanged. Table 2 shows the products from partial combustion of ethane in the reactor are principally ethylene and synthesis gas.

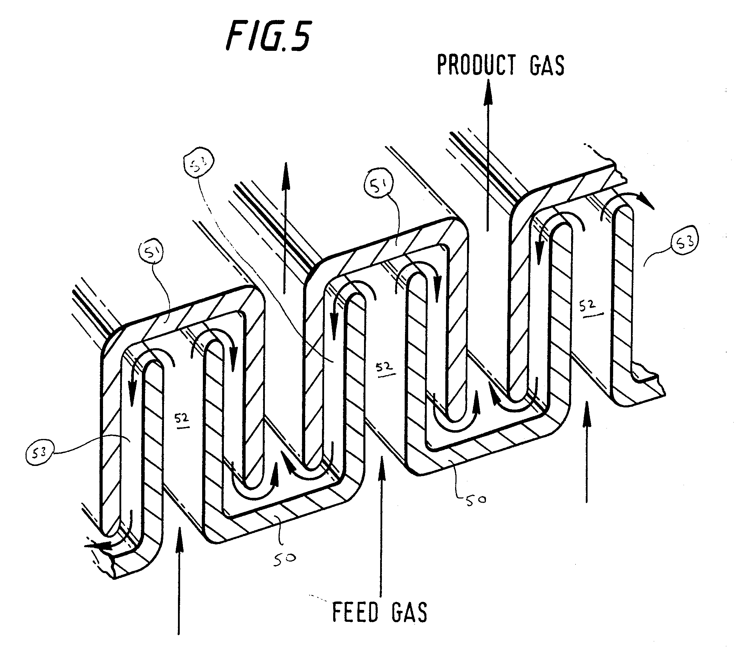

Figure 5 shows a part of a planar form of reactor. The figure shows three reaction chamber modules acting in parallel. The reaction chamber modules are formed from elongate channel section members 50, 51 which intermesh to form inlet zones 52 and outlet zones 53. The reaction products of each module pass form the outlet zones and are brought together above the reaction chambers for quenching.

**Claims**

1 A gas conversion reactor comprising a reaction chamber having an inlet zone leading to an outlet zone, the zones being adapted to allow indirect heat exchange from the outlet zone to the inlet zone, the inlet zone having an inlet for pre-mixed reactant gases and the reaction chamber having an outlet to a quenching zone.

2 A gas conversion reactor according to claim 1 in which the cross sectional area of the outlet zone is greater than the inlet zone.

3 A gas conversion reactor according to claim 2 in which the upstream end of the outlet zone has the same or a similar cross sectional area as the downstream end of the inlet zone, the outlet zone then diverging at least partway along its length.

4 A gas conversion reactor according to claim 2 in which the outlet zone has one or more steps, each step being of greater cross sectional area then the inlet zone.

5 A gas conversion reactor according to any of the preceding claims in which the reaction chamber has an inlet zone in the form of a central annulus or tube leading to the outlet zone in the form of an annular chamber, the central annulus and annular chamber having a common wall thereby allowing indirect heat exchange therebetween.

6 A gas conversion reactor according to any of claims 1 to 4 in which the reaction chamber takes the form of an interconnected planar array of inlet and outlet zones.

7 A gas conversion reactor according to claim 6 in which the interconnected planar array of inlet and outlet zones comprise first and second parallel arrays of elongate channel section members, the arrays being oriented to face each other and to be staggered with respect to each other, the channel section members of the arrays intermeshing to form the inlet and outlet zones.

8 A process for producing unsaturated hydrocarbons in which (a) pre-mixed saturated hydrocarbon gas and an oxygen containing gas having a stoichiometric ratio in excess of that required for complete combustion is passed into a reaction chamber (b) the reaction chamber being arranged to allow indirect heat exchange between the reactants and the reaction products (c) the hydrocarbon gas and oxygen containing gas are ignited and reacted together and (d) reaction products are quenched.

9 A process according to claim 8 in which the reactants are pre-heated prior to entry into the reaction chamber.

10 A process according to claim 8 or 9 in which the products of the reaction are quenched by a water spray.

11 A process according to claim 8 in which hydrogen gas is co-fed with the reactants into the reaction chamber.

0287380

**FIG.1**

**FIG.2**

0287380

FIG.3

FIG.4

0287380

# FIG.5

PRODUCT GAS

FEED GAS